# EUROPEAN PATENT SPECIFICATION

(11) **EP 2 690 045 B1**
(45) Date of publication and mention of the grant of the patent: **08.08.2018**
(21) Application number: 13173592.0
(22) Date of filing: 25.06.2013
(51) Int. Cl.: B65H 63/06, B65H 61/00, D01H 13/26, D01H 13/32, G01N 33/36

(54) **Yarn monitoring device, yarn winding unit, and yarn winding machine**
Garnüberwachungsvorrichtung, Garnwickeleinheit und Garnwickelmaschine
Dispositif de surveillance de fil, unité de bobinage de fil et machine de bobinage de fil

(30) Priority: 24.07.2012 JP 2012163914
(43) Date of publication of application: 29.01.2014
(73) Proprietor: Murata Machinery, Ltd., Minami-ku Kyoto-shi Kyoto 601-8326 (JP)
(72) Inventor: Nakatani, Masatoshi, Kyoto, 612-8686 (JP); Minamino, Katsushi, Kyoto, 612-8686 (JP)
(74) Representative: Zimmermann, Tankred Klaus

(56) References cited:
- EP-A1- 2 410 316
- EP-A2- 2 108 723
- US-A- 4 916 625

## Description

### BACKGROUND OF THE INVENTION

### 1. Field of the Invention

The present invention relates to a yarn monitoring device that monitors a running yarn, a yarn winding unit that includes the yarn monitoring device, and a yarn winding machine that includes the yarn winding unit.

### 2. Description of the Related Art

Yarn winding machines, such as automatic winders, that include a clearer that monitors a thickness of a running yarn are known in the art. Such a yarn winding machine that includes a clearer is disclosed, for example, in Japanese Patent Application Laid-open No. 2009-190841. The disclosed clearer has two yarn irregularity sensors arranged along a running direction of the yarn. This clearer detects whether there is an irregularity in a thickness of the yarn based on a detection result generated by one of the yarn irregularity sensors, and measures a running speed of the yarn based on signals generated in the two yarn irregularity sensors.

To satisfy the demand for faster processing of the signals by the clearer, one approach is to provide two signal processors in the clearer, one signal processor for detecting any irregularity in a thickness of the yarn and the other signal processor for measuring the running speed of the yarn. Even in a clearer having two signal processors, it is desirable to reduce the processing load on the signal processors to achieve still faster processing speed.

EP 2 108 723 A2 describes an automatic winder system having a plurality of automatic winders. Each of the automatic winders includes a plurality of yarn winding units, a frame control device, and a ZigBee terminal. The frame control device manages information on the yarn winding unit and controls the yarn winding unit. The ZigBee terminal can carry out wireless communication directly with a ZigBee terminal provided in another automatic winder (60).

EP 2 410 316 A1 describes a clearer for a yarn winding unit having a yellow light source section, a blue light source section, a first light receiving section, a second light receiving section, a first detecting section, and a second detecting section. first detecting section detects a foreign particle in the spun yarn, and the second detecting section detects a thickness of the spun yarn.

US 4 916 625 A describes method for monitoring the operation of a multiposition spinning machine. The operative state of each position is sensed by monitoring the presence or absence of the advancing yarn bundle and threadlines as a function of time and this information is provided to the computer which compares the operative state of each position and the elapsed time since initiation of each package and indicates that the time for performing said events has been reached.

### SUMMARY OF THE INVENTION

It is an object of the present invention to provide a yarn monitoring device, a yarn winding unit, and a yarn winding machine in which there is less processing load on signal processors.

This object is achieved by a yarn monitoring device according to claim 1, a yarn winding unit according to claim 8, and a yarn winding machine according to claim 9.

The above and other objects, features, advantages and the technical and industrial significance of this invention will be better understood by reading the following detailed description of presently preferred embodiments of the invention, when considered in connection with the accompanying drawings.

### BRIEF DESCRIPTION OF THE DRAWINGS

FIG. 1 is a side view of a winder unit included in an automatic winder according to an embodiment of the present invention;
FIG. 2 is a front elevational view of the winder unit shown in FIG. 1;
FIG. 3 is a drawing showing how each of the winder units shown in FIG. 1 is connected to a setting and monitoring device;
FIG. 4 is a block diagram of a clearer shown in FIG. 1; and
FIG. 5 is a flowchart of a process implemented when a second CPU of the clearer downloads an updated computer program.

### DETAILED DESCRIPTION

Exemplary embodiments of an automatic winder (yarn winding machine) that includes a yarn monitoring device and a yarn winding unit according to the present invention are explained in detail below with reference to the accompanying drawings.

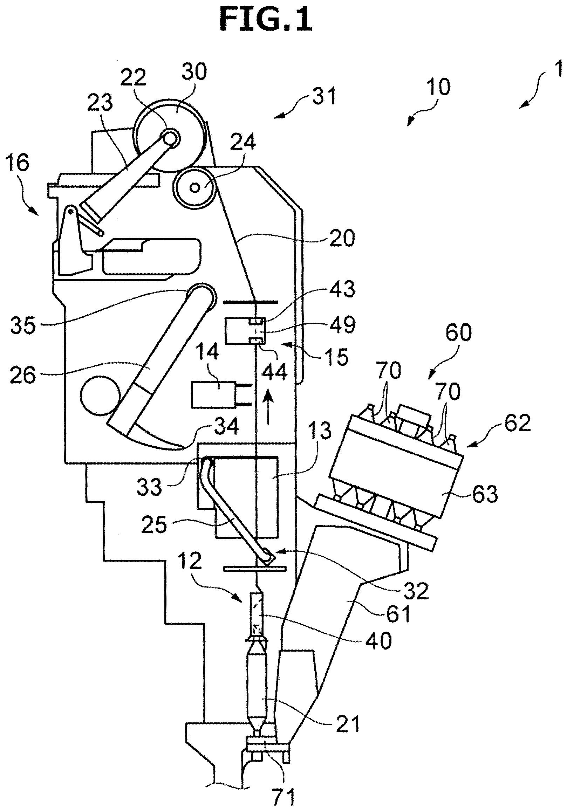

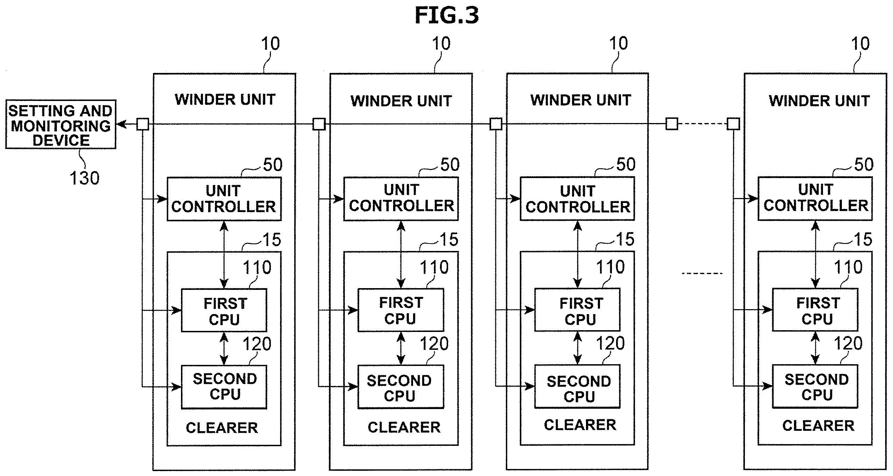

As shown in FIGS. 1 to 3, an automatic winder (yarn winding machine) 1 includes a plurality of winder units (yarn winding units) 10 that are aligned side by side, and a setting and monitoring device (first external device) 130 (see FIG. 3) that functions as a central control device arranged at one end in the alignment direction of the winder units 10. Each of the winder units 10 unwinds a spun yarn 20 from a supply bobbin 21 and winds the spun yarn 20 around a winding bobbin 22 while traversing the spun yarn 20 to form a package 30 of a predetermined shape. The setting and monitoring device 130 communicates with a clearer 15, a unit controller 50, and the like, which are arranged in each of the winder units 10, and performs settings and monitoring of operations of the respective winder unit 10.

Each of the winder units 10 includes a winding unit main body 16. The winding unit main body 16 includes a magazine-type supplying device 60, a winding section 31, and a supply bobbin holding section 71.

The magazine-type supplying device 60, as shown in FIG. 1, includes a magazine holding member 61 that extends diagonally upward from the bottom of the winder unit 10 in a machine height direction toward the front of the winder unit 10, and a bobbin housing device 62 that is coupled to the leading end of the magazine holding member 61. The bobbin housing device 62 includes a magazine can 63. The magazine can 63 has a plurality of housing holes in which supply bobbins 70 can be fitted. The magazine can 63 is intermittently driven to rotate by a not-shown motor, and by this intermittent driving and a not-shown regulating valve included in the magazine can 63, the supply bobbins 70 are dropped one by one into a not-shown bobbin supply path in the magazine holding member 61. The supply bobbin 70 is led to the supply bobbin holding section 71 in this manner.

A transport conveyor can be used instead of the magazine-type supplying device 60 shown in FIG. 1. The transport conveyor can be arranged at the bottom of the winder unit 10 in the machine height direction to supply the supply bobbin 21 from a not-shown supply bobbin supplying section to the supply bobbin holding section 71 of each winder unit 10.

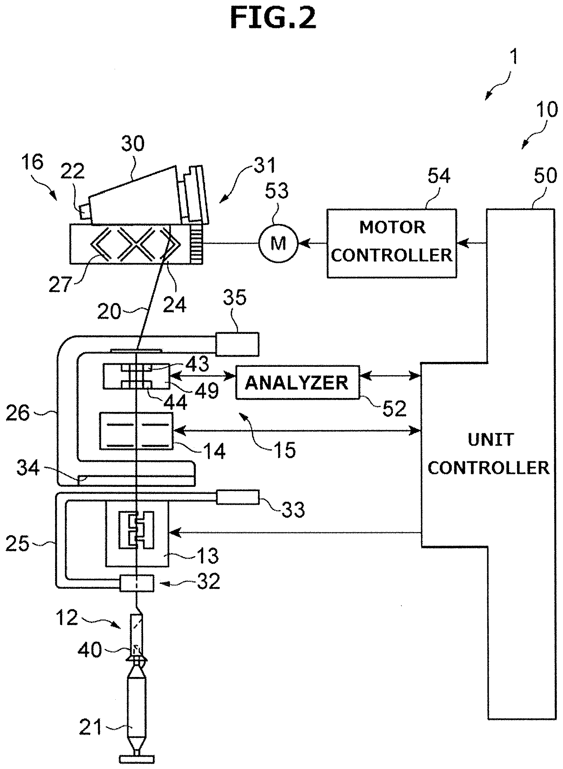

The winding section 31 unwinds the spun yarn 20 from the supply bobbin 21 and winds the spun yarn 20 around the winding bobbin 22 to form the package 30. Specifically, the winding section 31 includes a cradle 23 that holds the winding bobbin 22 and a winding drum 24 that traverses the spun yarn 20 and drives the winding bobbin 22. The cradle 23 swings in an approaching direction and a separating direction relative to the winding drum 24. Consequently, the package 30 comes into contact with or moves away from the winding drum 24. As shown in FIG. 2, traverse grooves 27 are formed on an outer peripheral surface of the winding drum 24. The winding drum 24 traverses the spun yarn 20 along those traverse grooves 27.

The winding bobbin 22 is driven to rotate when the winding drum 24, which is opposed to the winding bobbin 22, is driven to rotate. The spun yarn 20 is traversed by the traverse grooves 27 and wound around the rotating winding bobbin 22. As shown in FIG. 2, the winding drum 24 is coupled to an output shaft of a drum driving motor 53. The operation of the drum driving motor 53 is controlled by a motor controller 54. The motor controller 54 receives a control signal from the unit controller (second external device) 50, and exerts control to run and stop the drum driving motor 53.

In a yarn running path between the supply bobbin 21 and the winding drum 24 in the winding unit main body 16, an unwinding assisting device 12, a tension applying device 13, a yarn joining device 14, and a clearer head 49 included in the clearer (yarn monitoring device) 15 are arranged in the mentioned order from the supply bobbin 21 side.

The unwinding assisting device 12 assists the unwinding of the spun yarn 20 from the supply bobbin 21 by moving downward a regulating member 40 that caps the inner tube of the supply bobbin 21 as the spun yarn 20 is unwound from the supply bobbin 21. The regulating member 40 comes into contact with a balloon formed on the upper portion of the supply bobbin 21 due to the swaying of the spun yarn 20 unwound from the supply bobbin 21, applies an appropriate tension to the balloon, and thereby assists the unwinding of the spun yarn 20.

The tension applying device 13 applies a predetermined tension to the running spun yarn 20. The quality of the package 30 can be enhanced by application of a constant tension to the spun yarn 20 by the tension applying device 13.

The clearer 15 detects, as a quality check, whether there is a defect in the spun yarn 20. Whether there is a defect is decided by detecting irregularities (irregularity in the thickness) in the spun yarn 20. For this purpose, the clearer 15 includes the clearer head 49 and an analyzer 52 (see FIG. 2). A first yarn irregularity sensor 43 and a second yarn irregularity sensor 44 are arranged in the clearer head 49. The analyzer 52 detects yarn defects, such as a slub, by processing signals generated at the first yarn irregularity sensor 43 and the second yarn irregularity sensor 44. A not-shown cutter arranged near the clearer head 49 cuts the spun yarn 20 immediately after a yarn defect is detected by the clearer 15.

The clearer 15 also functions as a sensor that determines the running speed of the spun yarn 20. A structure in which the clearer 15 measures the running speed of the spun yarn 20 is explained later.

The yarn joining device 14 joins a lower yarn from the supply bobbin 21 and an upper yarn from the package 30 when the spun yarn 20 is cut due to detection of a yarn defect by the clearer 15 or when the spun yarn 20 runs out during unwinding from the supply bobbin 21. A mechanical-type yarn joining device or a yarn joining device that employs a fluid, such as compressed air, can be used as the yarn joining device 14.

A lower yarn guiding pipe 25 is arranged on the lower side of the yarn joining device 14 in the machine height direction of the winder unit 10; an upper yarn guiding pipe 26 is arranged on the upper side of the yarn joining device 14. The lower yarn guiding pipe 25 catches and guides the lower yarn from the supply bobbin 21; the upper yarn guiding pipe 26 catches and guides the upper yarn from the package 30. A suction vent 32 is formed at the leading end of the lower yarn guiding pipe 25. A suction mouth 34 is arranged at the leading end of the upper yarn guiding pipe 26. An appropriate negative pressure source (not shown) is coupled to each of the lower yarn guiding pipe 25 and the upper yarn guiding pipe 26 so that a suction flow is produced at the suction vent 32 and the suction mouth 34.

When the spun yarn 20 runs out or is cut, the suction vent 32 of the lower yarn guiding pipe 25 catches the lower yarn at the position shown in FIGS. 1 and 2, and thereafter swings upward about a shaft 33, thereby guiding the lower yarn to the yarn joining device 14. Substantially at the same time, the upper yarn guiding pipe 26 swings upward about a shaft 35 from the position shown in FIGS. 1 and 2, and catches the upper yarn unwound from the package 30 by the suction mouth 34. Thereafter, the upper yarn guiding pipe 26 swings downward about the shaft 35, thereby guiding the upper yarn to the yarn joining device 14. The yarn joining device 14 joins the guided lower and upper yarns.

The unit controller 50, as shown in FIG. 3, is provided in each of the winder units 10. The unit controller 50 controls the components of the winder unit 10 such as the drum driving motor 53, and/or performs various processes such as determination of a length of the spun yarn 20 wound in the package 30. The unit controller 50 manages address information that is required for the communication of a first central processing unit (CPU) 110 and a second CPU 120 arranged in the analyzer 52 with the setting and monitoring device 130. In response to a request from the first CPU 110, the unit controller 50 sends the address information of the first CPU 110 to the first CPU 110. In response to a request from the second CPU 120, the unit controller 50 sends the address information of the second CPU 120 to the second CPU 120 via the first CPU 110.

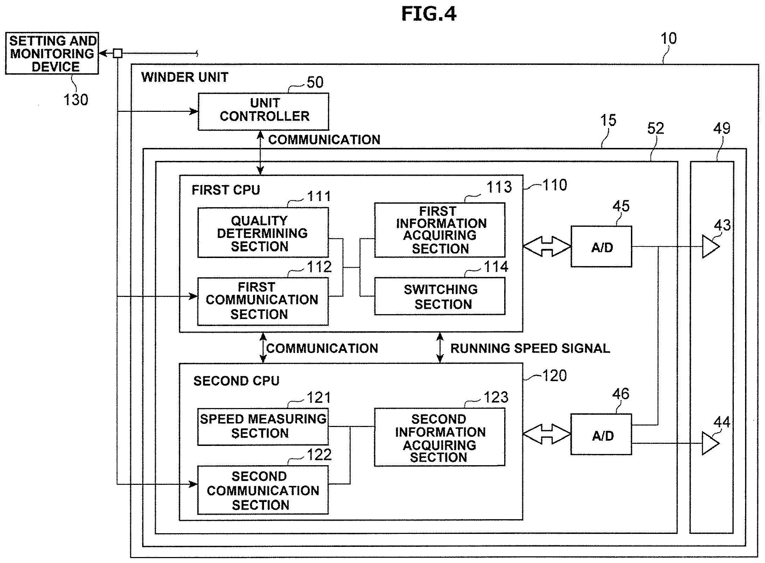

The clearer 15 is explained in greater details with reference to FIG. 4. As shown in FIG. 4, the clearer head 49 includes the first yarn irregularity sensor 43 and the second yarn irregularity sensor 44. The analyzer 52 includes the first CPU 110 that functions as a first signal processor, the second CPU 120 that functions as a second signal processor, a first A/D converter 45, and a second A/D converter 46. When a certain winder unit 10 is performing a winding operation to form the package 30, the clearer 15 of this winder unit 10 communicates with the setting and monitoring device 130 and the unit controller 50.

The first yarn irregularity sensor 43 and the second yarn irregularity sensor 44 are arranged along the running direction of the spun yarn 20 with a predetermined gap between them. The first yarn irregularity sensor 43 is arranged on the downstream side and the second yarn irregularity sensor 44 is arranged on the upstream side in the running direction of the spun yarn 20.

The first A/D converter 45 samples analog signals obtained from the first yarn irregularity sensor 43, and converts the analog signals into digital signals. The digital signals are input into the first CPU 110.

The second A/D converter 46 samples analog signals obtained from the first yarn irregularity sensor 43 and the second yarn irregularity sensor 44, and converts the analog signals into respective digital signals. The digital signals are input into the second CPU 120.

The first CPU 110 functions as a quality determining section (first processing section) 111, a first communication section 112, a first information acquiring section 113, and a switching section 114.

The quality determining section 111 determines the quality of the spun yarn 20 based on the digital signal received from the first A/D converter 45. Specifically, the quality determining section 111 detects the yarn irregularity as criteria for determining the quality of the spun yarn 20. When detecting the yarn irregularity, first, the quality determining section 111 continuously detects the thickness of the spun yarn 20 based on the digital signals that are input, thereby accumulating the detected thickness data. Based on the accumulated thickness data of a given length of the spun yarn 20, the quality determining section 111 determines presence/absence of a thickness irregularity in the form of the thickness of the spun yarn 20, such as a certain portion thereof being thicker or thinner than a predetermined value, and thereby detects the yarn irregularity. Furthermore, running speed signals of the spun yarn 20 calculated by the second CPU 120 are input into the quality determining section 111. The quality determining section 111 samples thickness data of the spun yarn 20 by every given (predetermined) length of the spun yarn 20, based on the received running speed signals.

The first communication section 112 is communicably connected to the unit controller 50 and the setting and monitoring device 130. The first communication section 112 constantly monitors whether a command is received from the setting and monitoring device 130. The first communication section 112 communicates with the setting and monitoring device 130 upon receiving such a command from the setting and monitoring device 130. The first communication section 112 can also start communication with the setting and monitoring device 130 by sending a communication start command to the setting and monitoring device 130. The first communication section 112 sends a yarn irregularity detection result and the like obtained by the quality determining section 111 to the setting and monitoring device 130 at appropriate timing.

The first communication section 112 sends the yarn irregularity detection result obtained from the quality determining section 111, information that the spun yarn 20 is running, and the like to the unit controller 50 at appropriate timing.

The first information acquiring section 113 is communicably connected to the unit controller 50. The first information acquiring section 113 acquires from the unit controller 50 the address information of the first CPU 110 that is to be used when communicating with the setting and monitoring device 130. The first information acquiring section 113 receives from the unit controller 50 the address information of the second CPU 120 that is to be used when communicating with the setting and monitoring device 130, and sends the received address information to the second CPU 120.

The switching section 114 issues, in response to an instruction from the setting and monitoring device 130, an instruction to the second CPU 120 so that the second CPU 120 switches between a communication-enabled state of being capable of communicating with the setting and monitoring device 130 and a communication-disabled state of being incapable of communicating with the setting and monitoring device 130.

The second CPU 120 functions as a speed measuring section (second processing section) 121, a second communication section 122, and a second information acquiring section 123.

The speed measuring section 121 measures the running speed of the spun yarn 20 based on the digital signals received from the second A/D converter 46. The speed measuring section 121 measures the running speed of the spun yarn 20 based on, for example, a time difference between a time when a certain portion of the spun yarn 20 runs past the second yarn irregularity sensor 44 and a time when it runs past the first yarn irregularity sensor 43, and also based on a distance between the first yarn irregularity sensor 43 and the second yarn irregularity sensor 44.

The second communication section 122 is communicably connected to the setting and monitoring device 130. The second communication section 122 is also communicably connected to the unit controller 50 via the first CPU 110. The second communication section 122 switches between the communication-enabled state and the communication-disabled state based on an instruction from the switching section 114. In the communication-disabled state, the second communication section 122 does not monitor the command sent from the setting and monitoring device 130 to the second communication section 122. In the communication-enabled state, the second communication section 122 monitors the command sent from the setting and monitoring device 130 to the second communication section 122. The second communication section 122 communicates with the setting and monitoring device 130 upon receiving such a command. The second communication section 122 is always in readiness to receive from the switching section 114 the instruction to switch the second CPU 120 between the communication-enabled state and the communication-disabled state.

The second communication section 122 outputs the running speed of the spun yarn 20 that is measured by the speed measuring section 121 to the first CPU 110. The second communication section 122 can also output the measured running speed of the spun yarn 20 to the unit controller 50. In this case, the unit controller 50 can determine the length of the spun yarn 20 in the formed package 30 based on the running speed signals of the spun yarn 20 that are output by the second CPU 120.

The second information acquiring section 123 acquires, from the unit controller 50 via the first CPU 110, the address information of the second CPU 120 that is used for communicating with the setting and monitoring device 130. The second information acquiring section 123 is always in readiness to receive the address information of the second CPU 120 from the first CPU 110.

A duration for which the first communication section 112 of the first CPU 110 communicates with the setting and monitoring device 130 (first duration) is longer than a duration for which the second communication section 122 of the second CPU 120 communicates with the setting and monitoring device 130 (second duration).

The setting and monitoring device 130 receives the yarn irregularity detection result from the first CPU 110. The setting and monitoring device 130 also communicates with the first CPU 110 and the second CPU 120 based on an instruction or the like from an operator of the automatic winder 1, and sends, for example, an updated computer program to the first CPU 110 and the second CPU 120.

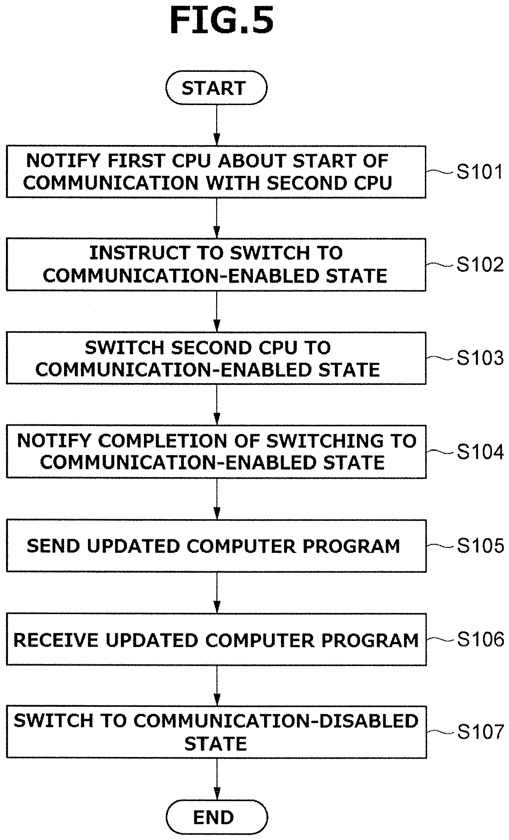

A flow of a process performed when the second CPU 120 downloads an updated computer program from the setting and monitoring device 130 is explained below. It is assumed here that the second information acquiring section 123 of the second CPU 120 has acquired the address information of the second CPU 120 from the unit controller 50 in advance. As shown in FIG. 5, the setting and monitoring device 130 notifies the first communication section 112 of the first CPU 110 about start of communication with the second CPU 120, in response to an instruction from the operator of the automatic winder 1 (Step S101).

The switching section 114 of the first CPU 110 issues an instruction to the second CPU 120 to switch to the communication-enabled state (Step S102). The second communication section 122 of the second CPU 120 switches to the communication-enabled state, in response to the instruction received from the switching section 114 (Step S103). The second communication section 122 of the second CPU 120 notifies the setting and monitoring device 130 via the first communication section 112 of the first CPU 110 about completion of switching to the communication-enabled state (Step S104).

Upon receiving the notification that the second communication section 122 of the second CPU 120 has been switched to the communication-enabled state, the setting and monitoring device 130 sends the updated computer program to the second communication section 122 of the second CPU 120, based on the address information assigned to the second CPU 120 (Step S105). The second communication section 122 of the second CPU 120 receives the updated computer program sent for the second CPU 120 by the setting and monitoring device 130 (Step S106). The second communication section 122 of the second CPU 120 therefore receives the updated computer program directly from the setting and monitoring device 130 instead of receiving it via the first CPU 110.

When the downloading of the updated computer program is completed, the second communication section 122 of the second CPU 120 switches to the communication-disabled state (Step S107). The second CPU 120, which has received the updated computer program, now performs an updating process of the computer program.

The first communication section 112 of the first CPU 110 is constantly monitoring the command sent from the setting and monitoring device 130 to the first CPU 110. Consequently, when downloading the updated computer program, the first CPU 110 is always in readiness to receive the updated computer program sent for the first CPU 110 from the setting and monitoring device 130.

With the above-explained structure of the present embodiment, the switching section 114 of the first CPU 110 switches the second communication section 122 of the second CPU 120 to the communication-enabled state as the occasion demands. Because the second communication section 122 of the second CPU 120 becomes, only when it is switched to the communication-enabled state by the switching section 114, capable of communicating with the setting and monitoring device 130, the processing load on the second CPU 120 is reduced. Accordingly, it is possible to provide the winder unit 10 that includes the clearer 15 in which the processing load on the second CPU 120 is reduced, and also the automatic winder 1 that includes such a winder unit 10.

The second information acquiring section 123 of the second CPU 120 acquires the address information of the second CPU 120 from the unit controller 50 via the first CPU 110. Consequently, wiring that directly connects the second CPU 120 and the unit controller 50 to send and receive address information is rendered unnecessary, and thereby a simple structure can be realized.

The quality determining section 111 of the first CPU 110 detects the yarn irregularity based on the signals obtained from the first yarn irregularity sensor 43. In the clearer 15 that includes the quality determining section 111 that determines the quality of the spun yarn 20, the processing load on the second CPU 120 can be reduced.

The first communication section 112 of the first CPU 110 sends the yarn irregularity detection result obtained from the quality determining section 111 to the setting and monitoring device 130. Consequently, the yarn irregularity detection result can be utilized by the setting and monitoring device 130 in addition to the clearer 15.

The speed measuring section 121 of the second CPU 120 measures the running speed of the spun yarn 20 based on the signals obtained from the first yarn irregularity sensor 43 and the second yarn irregularity sensor 44. Because the running speed of the spun yarn 20 does not need to be sent to the setting and monitoring device 130 all the time, the switching section 114 of the first CPU 110 switches the second CPU 120, which measures the running speed of the spun yarn 20, to the communication-enabled state only when the occasion demands. Consequently, in the clearer 15 that includes the first CPU 110 that detects the yarn irregularity and the second CPU 120 that measures the running speed of the spun yarn 20, the processing load on the second CPU 120 can be favorably reduced.

The duration for which the first CPU 110 communicates with the setting and monitoring device 130 is longer than the duration for which the second CPU 120 communicates with the setting and monitoring device 130. Consequently, the second CPU 120 with a shorter communication period can be switched to the communication-enabled state only when occasion demands, and thereby the processing load on the second CPU 120 can be reduced.

The second CPU 120 is communicably connected to the unit controller 50 via the first CPU 110. Consequently, wiring that directly connects the second CPU 120 and the unit controller 50 is rendered unnecessary, and thereby the winder unit 10 having a simple structure can be realized.

An embodiment of the present invention has been explained above; however, the present invention is not limited thereto. In the present embodiment, it has been assumed that when downloading the updated computer program from the setting and monitoring device 130, the second CPU 120 has already acquired the address information of the second CPU 120 in advance. Instead, when the setting and monitoring device 130 outputs the instruction to the first CPU 110 to switch the second CPU 120 to the communication-enabled state, the first information acquiring section 113 of the first CPU 110 can acquire the address information of the second CPU 120 from the unit controller 50 and output the address information to the second CPU 120.

In the present embodiment, an updated computer program has been described as an example of data sent from the setting and monitoring device 130 to the first CPU 110 and the second CPU 120. However, data other than the updated computer program, such as computer programs for yarn running speed detection or bug-corrected computer programs can also be sent.

In the present embodiment, the first communication section 112 of the first CPU 110 sends the yarn irregularity detection result to the setting and monitoring device 130. However, information other than the yarn irregularity detection result can be sent by the first communication section 112 to the setting and monitoring device 130. For example, the quality determining section 111 of the first CPU 110 calculates, as quality information of the spun yarn 20, at least one of residual defect information that is information pertaining to yarn defects that do not warrant cutting but warrant notifying, information pertaining to the number of yarn defects per unit length that do not warrant cutting but warrant notifying, and information pertaining to the number of yarn irregularity components per unit length. The first communication section 112 of the first CPU 110 can also send information calculated by the quality determining section 111 to the setting and monitoring device 130.

In the present embodiment, the speed measuring section 121 of the second CPU 120 measures the running speed of the spun yarn 20; however, the second CPU 120 can measure and obtain information other than the running speed. For example, the second CPU 120 can obtain presence/absence of yarn irregularities that periodically appear. The obtained information pertaining to the presence/absence of periodically appeared yarn irregularity can be sent to the unit controller 50 or to the setting and monitoring device 130 at appropriate timing. The second communication section 122 of the second CPU 120 can also send the information pertaining to the presence/absence of periodically appeared yarn irregularity to the setting and monitoring device 130 via the first CPU 110. Alternatively, the second communication section 122 of the second CPU 120 can send, upon receiving an instruction from the switching section 114 of the first CPU 110 to switch to the communication-enabled state, the information pertaining to the presence/absence of periodically appeared yarn irregularity directly from the second CPU 120 to the setting and monitoring device 130.

In the present embodiment, the switching section 114 of the first CPU 110 instructs the second CPU 120 to switch between the communication-enabled state and the communication-disabled state. Alternatively, the second communication section 122 of the second CPU 120 can be switched between the communication-enabled state and the communication-disabled state with the setting and monitoring device 130 by using an I/O port of the first CPU 110 and an I/O port of the second CPU 120 to turn the communication on and off.

In the present embodiment, the first A/D converter 45 and the second A/D converter 46 are provided separately from the first CPU 110 and the second CPU 120, respectively, in the clearer 15. However, the A/D converters can be provided inside the first CPU 110 and the second CPU 120.

The use of the clearer 15 that includes the first CPU 110 and the second CPU 120 is not limited to the automatic winder 1 alone; it can also be used in other yarn winding machines.

According to an aspect of the present embodiment, a yarn monitoring device includes a first signal processor and a second signal processor. The first signal processor includes a first processing section that processes a signal pertaining to a running yarn, a first communication section that is capable of communicating with a first external device, and a switching section that switches the second signal processor between a state of being capable of communicating with the first external device and a state of being incapable of communicating with the first external device. The second signal processor includes a second processing section that processes the signal pertaining to the running yarn differently from the first processing section, and a second communication section that is capable of communicating with the first external device.

In this yarn monitoring device, the switching section of the first signal processor switches the second signal processor to the state, i.e., a communication-enabled state, in which it is capable of communicating with the first external device as the occasion demands. Because the second signal processor becomes, only when it is switched to the communication-enabled state by the switching section of the first signal processor, capable of communicating with the first external device, a processing load on the second signal processor can be reduced.

In the yarn monitoring device according to another aspect of the present embodiment, the first signal processor includes a first information acquiring section that acquires, from a second external device, address information of the first signal processor for communicating with the first external device. The second signal processor includes a second information acquiring section that acquires, from the second external device via the first signal processor, address information of the second signal processor for communicating with the first external device. Wiring that directly connects the second signal processor and the second external device is rendered unnecessary, and thereby a simple structure can be realized.

In the yarn monitoring device according to still another aspect of the present embodiment, the first processing section determines a yarn quality based on the signal. Consequently, in the yarn monitoring device that includes the first processing section that determines the quality of the yarn, the processing load on the second signal processor can be reduced.

In the yarn monitoring device according to still another aspect of the present embodiment, the first communication section sends a determination result of the yarn quality determined by the first processing section to the first external device. Consequently, the determination result of the quality of the yarn can be utilized by the first external device in addition to the yarn monitoring device.

In the yarn monitoring device according to still another aspect of the present embodiment, the second processing section measures a yarn running speed based on the signal. Because the running speed of the yarn does not need to be sent to the first external device all the time, the first signal processor switches the second signal processor that measures the running speed of the yarn to the communication-enabled state only when the occasion demands. Consequently, in the yarn monitoring device that includes the second signal processor that measures the running speed of the yarn, the processing load on the second signal processor can be more favorably reduced.

In the yarn monitoring device according to still another aspect of the present embodiment, a first duration for which the first signal processor communicates with the first external device is longer than a second duration for which the second signal processor communicates with the first external device. The processing load on the second signal processor that has a shorter communication period can thereby be reduced.

According to still another aspect of the present embodiment, a yarn winding unit includes a second external device and the above yarn monitoring device. The first signal processor is communicably connected to the second external device. The second signal processor is communicably connected to the second external device via the first signal processor. Consequently, wiring that directly connects the second signal processor and the second external device is rendered unnecessary, and thereby a yarn winding unit having a simple structure can be realized.

According to still another aspect of the present embodiment, a yarn winding machine includes a plurality of the yarn winding units and the first external device. The first external device preferably performs at least one of setting of operations of the yarn winding units and monitoring of the operations of the yarn winding units. The first signal processor switches the second signal processor to the communication-enabled state as the occasion demands. Because the second signal processor becomes, only when it is switched to the communication-enabled state by the switching section of the first signal processor, capable of communicating with the first external device, a processing load on the second signal processor can be reduced.

According to the present invention, a processing load on the signal processors can be reduced.

## Claims

1. A yarn monitoring device comprising:
a first signal processor (110) that includes
a first processing section (111) adapted to process a signal pertaining to a running yarn (20), and
a first communication section (112) adapted to be capable of communicating with a first external device (130), wherein the first external device (130) is adapted to perform at least one of setting of an operation of a yarn winding unit (10) of a yarn winding machine and monitoring of the operation of the yarn winding unit (10); and
a second signal processor (120) that includes
a second processing section (121) adapted to process the signal pertaining to the running yarn (20) differently from the first processing section (111), and
a second communication section (122) adapted to be capable of communicating with the first external device (130);
**characterized in that**
the first signal processor (110) includes a switching section (114) adapted to switch the second signal processor (120) between a state of being capable of communicating with the first external device (130) and a state of being incapable of communicating with the first external device (130),
the first communication section (112) is configured to constantly monitor whether a command is received from the first external device (130), and
in the communication-disabled state, the second communication section (122) is configured to not monitor whether a command is sent from the first external device (130) to the second communication section (122).

2. The yarn monitoring device according to Claim 1, comprising:
a first yarn irregularity sensor (43); and
a second yarn irregularity sensor (44),
wherein the first yarn irregularity sensor (43) and the second yarn irregularity sensor (44) are arranged along the running direction of the running yarn (20) with a predetermined gap between them,
wherein the first processing section (111) is adapted to process signals obtained from the first yarn irregularity sensor (43), and
wherein the second processing section (121) is adapted to process signals obtained from the first yarn irregularity sensor (43) and the second yarn irregularity sensor (44).

3. The yarn monitoring device according to any one of Claim 1 or 2, wherein the first processing section (111) is adapted to determine a yarn quality based on the signal.

4. The yarn monitoring device according to Claim 3, wherein the first communication section (112) is adapted to send a determination result of the yarn quality determined by the first processing section (110) to the first external device (130).

5. The yarn monitoring device according to any one of Claims 1 to 4, wherein the second processing section (121) is adapted to measure a yarn running speed based on the signal.

6. The yarn monitoring device according to any one of Claims 1 to 5, wherein a first duration for which the first signal processor (110) communicates with the first external device (130) is longer than a second duration for which the second signal processor (120) communicates with the first external device (130).

7. The yarn monitoring device according to any one of Claims 1 to 6, wherein
the first signal processor (110) includes a first information acquiring section (113) adapted to acquire, from a second external device (50), address information of the first signal processor (110) for communicating with the first external device (130), wherein the second external device (50) is a unit controller of the yarn winding unit (10), and
the second signal processor (120) includes a second information acquiring section (123) adapted to acquire, from the second external device (50) via the first signal processor (110), address information of the second signal processor (120) for communicating with the first external device (130).

8. A yarn winding unit comprising:
a yarn monitoring device (15) according to Claim 7; and
the second external device (50),
wherein the first signal processor (110) is communicably connected to the second external device (50), and
the second signal processor (120) is communicably connected to the second external device (50) via the first signal processor (110).

9. A yarn winding machine comprising:
a plurality of the yarn winding units (10) according to Claim 8; and
the first external device (130).

## Patentansprüche

1. Eine Garnüberwachungsvorrichtung, die folgende Merkmale aufweist:
einen ersten Signalprozessor (110), der Folgendes umfasst:
einen ersten Verarbeitungsabschnitt (111), der angepasst ist, ein Signal zu verarbeiten, das zu einem laufenden Garn (20) gehört, und
einen ersten Kommunikationsabschnitt (112), der angepasst ist, mit einer ersten externen Vorrichtung (130) kommunizieren zu können, wobei die erste externe Vorrichtung (130) angepasst ist, ein Festlegen eines Vorgangs einer Garnwickeleinheit (10) einer Garnwickelmaschine und/oder ein Überwachen des Vorgangs der Garnwickeleinheit (10) durchzuführen; und
einen zweiten Signalprozessor (120), der Folgendes umfasst:
einen zweiten Verarbeitungsabschnitt (121), der angepasst ist, das Signal, das zu dem laufenden Garn (20) gehört, anders als der erste Verarbeitungsabschnitt (111) zu verarbeiten, und
einen zweiten Kommunikationsabschnitt (122), der angepasst ist, mit der ersten externen Vorrichtung (130) kommunizieren zu können;
**dadurch gekennzeichnet, dass**
der erste Signalprozessor (110) einen Schaltabschnitt (114) umfasst, der angepasst ist, den zweiten Signalprozessor (120) zwischen einem Zustand, in dem derselbe mit der ersten externen Vorrichtung (130) kommunizieren kann, und einem Zustand zu schalten, in dem derselbe nicht mit der ersten externen Vorrichtung (130) kommunizieren kann,
der erste Kommunikationsabschnitt (112) konfiguriert ist, konstant zu überwachen, ob ein Befehl von der ersten externen Vorrichtung (130) empfangen wird, und
der zweite Kommunikationsabschnitt (122) in dem kommunikationsdeaktivierten Zustand konfiguriert ist, nicht zu überwachen, ob ein Befehl von der ersten externen Vorrichtung (130) an den zweiten Kommunikationsabschnitt (122) gesendet wird.

2. Die Garnüberwachungsvorrichtung gemäß Anspruch 1, die folgende Merkmale aufweist:
einen ersten Garnunregelmäßigkeitssensor (43) und
einen zweiten Garnunregelmäßigkeitssensor (44),
wobei der erste Garnunregelmäßigkeitssensor (43) und der zweite Garnunregelmäßigkeitssensor (44) entlang der Laufrichtung des laufenden Garns (20) mit einem vorbestimmten Zwischenraum zwischen denselben angeordnet sind,
wobei der erste Verarbeitungsabschnitt (111) angepasst ist, Signale zu verarbeiten, die von dem ersten Garnunregelmäßigkeitssensor (43) erhalten werden, und
wobei der zweite Verarbeitungsabschnitt (121) angepasst ist, Signale zu verarbeiten, die von dem ersten Garnunregelmäßigkeitssensor (43) und dem zweiten Garnunregelmäßigkeitssensor (44) erhalten werden.

3. Die Garnüberwachungsvorrichtung gemäß einem der Ansprüche 1 oder 2, bei der der erste Verarbeitungsabschnitt (111) angepasst ist, eine Garnqualität auf Basis des Signals zu bestimmen.

4. Die Garnüberwachungsvorrichtung gemäß Anspruch 3, bei der der erste Kommunikationsabschnitt (112) angepasst ist, ein Bestimmungsergebnis der Garnqualität, das durch den ersten Verarbeitungsabschnitt (110) bestimmt wird, an die erste externe Vorrichtung (130) zu senden.

5. Die Garnüberwachungsvorrichtung gemäß einem der Ansprüche 1 bis 4, bei der der zweite Verarbeitungsabschnitt (121) angepasst ist, eine Garnlaufgeschwindigkeit auf Basis des Signals zu messen.

6. Die Garnüberwachungsvorrichtung gemäß einem der Ansprüche 1 bis 5, bei der ein erster Zeitraum, in dem der erste Signalprozessor (110) mit der ersten externen Vorrichtung (130) kommuniziert, länger als ein zweiter Zeitraum ist, in dem der zweite Signalprozessor (120) mit der ersten externen Vorrichtung (130) kommuniziert.

7. Die Garnüberwachungsvorrichtung gemäß einem der Ansprüche 1 bis 6, bei der
der erste Signalprozessor (110) einen ersten Informationsgewinnungsabschnitt (113) umfasst, der angepasst ist, von einer zweiten externen Vorrichtung (50) Adressinformationen des ersten Signalprozessors (110) zum Kommunizieren mit der ersten externen Vorrichtung (130) zu gewinnen, wobei die zweite externe Vorrichtung (50) eine Einheitssteuerung der Garnwickeleinheit (10) ist, und
der zweite Signalprozessor (120) einen zweiten Informationsgewinnungsabschnitt (123) umfasst, der angepasst ist, von der zweiten externen Vorrichtung (50) über den ersten Signalprozessor (110) Adressinformationen des zweiten Signalprozessors (120) zum Kommunizieren mit der ersten externen Vorrichtung (130) zu gewinnen.

8. Eine Garnwickeleinheit, die folgende Merkmale aufweist:
eine Garnüberwachungsvorrichtung (15) gemäß Anspruch 7 und
die zweite externe Vorrichtung (50),
wobei der erste Signalprozessor (110) mit der zweiten externen Vorrichtung (50) kommunizierend verbunden ist und
der zweite Signalprozessor (120) mit der zweiten externen Vorrichtung (50) über den ersten Signalprozessor (110) kommunizierend verbunden ist.

9. Eine Garnwickelmaschine, die folgende Merkmale aufweist:
eine Mehrzahl der Garnwickeleinheiten (10) gemäß Anspruch 8 und
die erste externe Vorrichtung (130).

## Revendications

1. Dispositif de surveillance de fil, comprenant:
un premier processeur de signal (110) qui comporte
un premier segment de traitement (111) adapté pour traiter un signal relatif à un fil en défilement (20), et
un premier segment de communication (112) adapté de manière à être à même de communiquer avec un premier dispositif externe (130), où le premier dispositif externe (130) est adapté pour effectuer un réglage d'un fonctionnement d'une unité de bobinage de fil (10) d'une machine de bobinage de fil et/ou une surveillance d'un fonctionnement de l'unité de bobinage de fil (10); et
un deuxième processeur de signal (120) qui comporte
un deuxième segment de traitement (121) adapté pour traiter le signal relatif au fil en défilement (20) de manière différente du premier segment de traitement (111), et
un deuxième segment de communication (122) adapté de manière à être à même de communiquer avec le premier dispositif externe (130);
**caractérisé par le fait que**
le premier processeur de signal (110) comporte un segment de commutation (114) adapté pour commuter le deuxième processeur de signal (120) entre un état où il est à même de communiquer avec le premier dispositif externe (130) et un état où il n'est pas à même de communiquer avec le premier dispositif externe (130),
le premier segment de communication (112) est configuré pour surveiller en permanence si une commande est reçue du premier dispositif externe (130), et
à l'état de désactivation de communication, le deuxième segment de communication (122) est configuré pour ne pas surveiller si une commande est envoyée du premier dispositif externe (130) au deuxième segment de communication (122).

2. Dispositif de surveillance de fil selon la revendication 1, comprenant:
un premier capteur d'irrégularité de fil (43); et
un deuxième capteur d'irrégularité de fil (44),
dans lequel le premier capteur d'irrégularité de fil (43) et le deuxième capteur d'irrégularité de fil (44) sont disposés le long de la direction de défilement du fil en défilement (20) avec un interstice prédéterminé entre eux,
dans lequel le premier segment de traitement (111) est adapté pour traiter les signaux obtenus du premier capteur d'irrégularité de fil (43), et
dans lequel le deuxième segment de traitement (121) est adapté pour traiter les signaux obtenus du premier capteur d'irrégularité de fil (43) et du deuxième capteur d'irrégularité de fil (44).

3. Dispositif de surveillance de fil selon l'une quelconque des revendications 1 ou 2, dans lequel le premier segment de traitement (111) est adapté pour déterminer une qualité de fil sur base du signal.

4. Dispositif de surveillance de fil selon la revendication 3, dans lequel le premier segment de communication (112) est adapté pour envoyer un résultat de détermination de la qualité de fil déterminé par le premier segment de traitement (110) au premier dispositif externe (130).

5. Dispositif de surveillance de fil selon l'une quelconque des revendications 1 à 4, dans lequel le deuxième segment de traitement (121) est adapté pour mesurer une vitesse de défilement de fil sur base du signal.

6. Dispositif de surveillance de fil selon l'une quelconque des revendications 1 à 5, dans lequel une première durée pendant laquelle le premier processeur de signal (110) communique avec le premier dispositif externe (130) est plus longue qu'une deuxième durée pendant laquelle le deuxième processeur de signal (120) communique avec le premier dispositif externe (130).

7. Dispositif de surveillance de fil selon l'une quelconque des revendications 1 à 6, dans lequel
le premier processeur de signal (110) comporte un premier segment d'acquisition d'informations (113) adapté pour acquérir, d'un deuxième dispositif externe (50), des informations d'adresse du premier processeur de signal (110) pour communiquer avec le premier dispositif externe (130), dans lequel le deuxième dispositif externe (50) est un dispositif de commande de l'unité de bobinage de fil (10), et
le deuxième processeur de signal (120) comporte un deuxième segment d'acquisition d'informations (123) adapté pour acquérir, du deuxième dispositif externe (50) par l'intermédiaire du premier processeur de signal (110), des informations d'adresse du deuxième processeur de signal (120) pour communiquer avec le premier dispositif externe (130).

8. Unité de bobinage de fil, comprenant:
un dispositif de surveillance de fil (15) selon la revendication 7; et
le deuxième dispositif externe (50),
dans lequel le premier processeur de signal (110) est connecté de manière à pouvoir communiquer au deuxième dispositif externe (50), et
le deuxième processeur de signal (120) est connecté de manière à pouvoir communiquer au deuxième dispositif externe (50) par l'intermédiaire du premier processeur de signal (110).

9. Machine de bobinage de fil, comprenant:
une pluralité d'unités de bobinage de fil (10) selon la revendication 8; et
le premier dispositif externe (130).
